# EUROPÄISCHE PATENTSCHRIFT

(11) **EP 2 285 481 B1**
(45) Veröffentlichungstag und Bekanntmachung des Hinweises auf die Patenterteilung: **11.07.2012**
(21) Anmeldenummer: 09765703.5
(22) Anmeldetag: 20.05.2009
(51) Int. Cl.: B01J 8/04, B01J 8/22, B01J 19/24

(54) **KONTINUIERLICHES VERFAHREN ZUR HYDRIERUNG ORGANISCHER VERBINDUNGEN**
CONTINUOUS METHOD FOR HYDROGENATING ORGANIC COMPOUNDS
PROCÉDÉ CONTINU D'HYDROGÉNATION DE COMPOSÉS ORGANIQUES

(30) Priorität: 27.05.2008 EP 08156991
(43) Veröffentlichungstag der Anmeldung: 23.02.2011
(73) Patentinhaber: BASF SE, 67056 Ludwigshafen (DE)
(72) Erfinder: PFEFFINGER, Joachim, 67071 Ludwigshafen (DE)
(86) Internationale Anmeldenummer: PCT/EP2009/056120
(87) Internationale Veröffentlichungsnummer: WO 2009/153123

(56) Entgegenhaltungen:
- EP-A- 0 010 571
- EP-A- 0 985 446
- DE-A1- 10 119 135

## Beschreibung

Die vorliegende Erfindung betrifft ein kontinuierliches Verfahren zur Hydrierung organischer Verbindungen in einem mehrphasigen System mit gelöstem oder suspendierten Katalysator, dadurch gekennzeichnet, dass man das Verfahren in zwei Stufen durchführt, wobei die erste Stufe in einem Schlaufenreaktor mit externem Wärmetauscher und die zweite Stufe in einem Blasensäulenreaktor mit begrenzter Rückmischung durchgeführt wird. Weiterhin betrifft die vorliegende Erfindung einen Reaktor, in dem eine erfindungsgemäße zweistufige Hydrierung durchgeführt werden kann.

Katalytische Hydrierungen organischer Verbindungen werden in der Regel an heterogenen Katalysatoren durchgeführt, wobei der Katalysator häufig als Festbett angeordnet ist. Bei der Hydrierung ungesättigter Verbindungen werden in der Regel beträchtliche Wärmemengen frei, wodurch die Festbettreaktoren mit teils sehr komplexen Einrichtungen zur Wärmeabfuhr ausgestattet werden müssen. Besteht noch zusätzlich die Aufgabe, die Hydrierung mit möglichst vollständigem Umsatz durchzuführen, müssen mehrere gekühlte Festbettreaktoren hintereinander geschaltet werden. Bei Hydrierungen kann es bereits nach kurzer Betriebszeit zu einem Nachlassen der Aktivität und/oder der Selektivität des Katalysators kommen, was einen Austausch oder, falls möglich, eine Regenerierung des Katalysators erforderlich macht. Bei einem Festbettreaktor kann dieser Katalysatoraustausch bzw. die Regenerierung im Allgemeinen nicht im laufenden Betrieb durchgeführt werden, so dass die Abstellung der Hydrieranlage erforderlich wird.

Alternativ kann eine heterogen katalytische Hydrierung in Form einer Suspensionsreaktion durchgeführt werden, wobei der Hydrierkatalysator durch Zufuhr mechanischer Energie z. B. in einem Rührkessel oder in einem Schlaufenreaktor suspendiert wird, vgl. z. B. Ullmann's Encyclopedia of Industrial Chemistry, Verlag Chemie, Electronic Edition Release 2008, 7th Edition, Kaptitel "Reactor Types and Their Industrial Application", Seite 24, Figur 11.

Die Herstellung von cycloaliphatischen Verbindungen durch Hydrierung von aromatischen Verbindungen mit suspendiertem Katalysator ist in der Literatur in zahlreichen Beispielen beschrieben, wie DE-C1-907294, US 2,606,925, DE-A1-2132547, US 4,394,522, US 4,448,995 oder DE-A1-3226889. Aus diesen Beispielen geht hervor, dass die Temperatur bei der Reaktion sowohl die Isomerenverteilung als auch die Bildung von Nebenprodukten beeinflussen kann.

Zur technischen Umsetzung von Hydrierungen finden sich in der Literatur ebenfalls zahlreiche Beispiele.

In DE-A1-1793452 wird ein kontinuierliches Verfahren zur Durchführung von katalytischen Hydrierungen beschrieben, bei welchem ein Teil des flüssigen Reaktionsmediums aus dem Reaktor abgezogen, durch einen Kühler gefahren und anschließend mit hoher Geschwindigkeit zusammen mit dem Wasserstoff unterhalb der Flüssigkeitsoberfläche wieder dem Reaktor zugeführt wird. Die Verweilzeitverteilung eines solchen Reaktors entspricht der Verweilzeitverteilung eines vollständig rückvermischten Rührkessels ("CSTR").

DE-A1-19647126 beschreibt einen mehrstufigen Blasensäulenreaktor mit eingebauten Lochplatten zur Kaskadierung des Reaktors.

EP-A2-57 364 offenbart einen Reaktor mit eingebauten Zwischenböden zur Hydrierung von Kohle bei hohem Druck, wobei durch die Zwischenböden die Rückvermischung im Reaktor verhindert werden soll.

EP-A2-985446 beschreibt ein zweistufiges, kontinuierliches Verfahren zur Hydrierung von Glukose zu Sorbitol, wobei die erste Stufe in einem Rührbehälter und die zweite Stufe in einem Blasensäulenreaktor erfolgt. Mit dieser Kombination kann die Verweilzeit so angepasst werden, dass eine Umsetzung des Einsatzstoffes bis zu 99,1% möglich wird.

DE-A1-10119135 beschreibt ein kontinuierliches Verfahren zur Herstellung von Diaminodicyclohexylmethan in einem Reaktorsystem mit mehreren hintereinander geschalteten Suspensionsreaktoren. Dabei werden sowohl Rührkesselreaktoren als auch Blasensäulenreaktoren als mögliche Einzelreaktoren beschrieben.

DE-A1-2039818 offenbart ein Verfahren und eine Reaktorverschaltung zur zweistufigen Hydrierung von Diaminodiphenylmethanen. Dabei wird die Umsetzung zunächst in einem Kreislaufreaktor mit suspendiertem Katalysator durchgeführt, der Katalysator abgetrennt und in den Kreislaufreaktor zurückgeführt und die Flüssigkeit einem zweiten Reaktor mit fest angeordnetem Katalysator zur vollständigen Umsetzung zugeführt.

Aus EP-A 0010571 ist ein zweiteiliger Reaktor aus einem Schlaufenreaktor und nachgeschaltetem Rohr bekannt. Hydrierverfahren werden nicht beschrieben.

Aus den aufgeführten Dokumenten geht hervor, dass die Temperatur bei der Hydrierung von organischen Verbindungen, insbesondere bei der Hydrierung von aromatischen Verbindungen, sowohl die Nebenproduktbildung als auch das Isomerenverhältnis beeinflussen kann. Bei zu niedrigen Temperaturen besteht im Allgemeinen die Gefahr, dass die Reaktion nicht anspringt bzw. zu langsam abläuft, während bei zu hohen Temperaturen es zum Ablauf unerwünschter Nebenreaktionen bis hin zum Durchgehen der Reaktion kommen kann.

Der Umsatz bei der Hydrierung sollte zudem möglichst vollständig sein, speziell bei der Hydrierung aromatischer Verbindungen zu den entsprechenden cycloaliphatischen Verbindungen, da ansonsten die nachfolgende Reinigung des erhaltenen Reaktions gemisches, beispielsweise durch Destillation, erschwert werden kann, da nicht umgesetzte Ausgangsprodukte schwer abtrennbare und schwer handhabbare Nebenprodukte bei der Aufarbeitung bilden können.

Der Stand der Technik offenbart einerseits die Verwendung von Reaktoren mit guter Durchmischung zur kontinuierlichen Hydrierung von aromatischen Verbindungen, die eine Verweilzeitverteilung eines kontinuierlichen Rührkesselreaktors aufweisen.
Die Verweilzeitverteilung in solchen Reaktoren bedingt, dass ein hoher Umsatz bei der Hydrierung in einem Rührkesselreaktor nur im absatzweisen Betrieb oder bei kontinuierlicher Fahrweise praktisch nur bei geringen Volumenströmen erreicht werden kann. Auch ist die Wärmeabfuhr an einem Rührbehälter, insbesondere bei großem Volumen, nur unzureichend möglich.
Andererseits werden im Stand der Technik Blasensäulenreaktoren als kontinuierliche Hydrierreaktoren offenbart.
Durch die Verwendung von Blasensäulenreaktoren, die mit Einbauten versehen sind, die die Rückvermischung begrenzen, kann die Verweilzeit verlängert und damit der Umsatz im Verfahren erhöht werden. Die Abführung der Reaktionswärme in Blasensäulenreaktoren ist allerdings, ähnlich wie bei Rührkesselreaktoren, begrenzt.

Durch die Kombination von Rührkesselreaktoren und Blasensäulenreaktor kann im kontinuierlichen Betrieb ein hoher Umsatz erzielt werden. Das Problem der Wärmeabfuhr im Hauptreaktionsteil (Rührkessel) des Reaktors wird allerdings nicht gelöst. Zudem ist die technisch Ausführung solcher Verfahren sehr aufwändig und damit kostenintensiv.

Die Hintereinanderschaltung von mehreren Blasensäulenreaktoren oder Rührkesselreaktoren zu einer Kaskade ermöglicht zwar das Erzielen von höheren Umsätzen im kontinuierlichen Betrieb, aber das Problem der Wärmeabfuhr bei exothermen Reaktionen wird durch diese Art von Reaktionsführung auch nicht gelöst. Zudem erhöhen sich die Investitionskosten, die Komplexität der Verfahrensführung und der technische Aufwand bei einer Kopplung von mehreren Reaktoren.

Die Kopplung von einem Schlaufenreaktor und einem Rohrreaktor, mit festem Katalysatorbett, erfordert die technisch aufwändige und teure Abtrennung von suspendiertem Katalysator nach der ersten Verfahrensstufe.

Der vorliegenden Erfindung lag deshalb die Aufgabe zu Grunde, ein technisch einfaches Verfahren zur Hydrierung von organischen Verbindungen zu entwickeln, mit welchem auch stark exotherme Hydrierungen in einem engen Temperaturbereich, mit hohem Durchsatz und mit sehr hohem Endumsatz in kontinuierlicher Fahrweise durchgeführt werden können. Durch das erfindungsgemäße Verfahren sollte es ermöglicht werden die Reaktion in einem definierten Temperaturbereich auszuführen um somit gezielt das Isomerenverhältnis der Edukte zu steuern sowie die Bildung von unerwünschten Nebenprodukten zu vermeiden. Das Verfahren sollte einen hohen Umsatz ermöglichen, um die Abtrennung von nicht umgesetzten Edukten zu vereinfachen. Weiterhin sollten die Reaktionsführung und die apparative Umsetzung des Verfahrens technisch einfach und wirtschaftlich zu realisieren sein. Weiterhin sollte dieses Verfahren lange Standzeiten ermöglichen, so dass die Anzahl von unerwünschten Abschaltungen der Reaktoren zum Austausch oder der Regeneration des Katalysators verringert werden können.

Erfindungsgemäß wurde die Aufgabe durch ein kontinuierliches Verfahren zur Hydrierung organischer Verbindungen in einem mehrphasigen System in Gegenwart eines homogenen oder heterogenen Katalysators, dadurch gekennzeichnet, dass man das Verfahren in zwei Stufen durchführt, wobei die erste Stufe in einem Schlaufenreaktor mit externem Wärmetauscher und die zweite Stufe in einem Blasensäulenreaktor mit begrenzter Rückmischung durchgeführt wird, gelöst.

Die vorliegende Erfindung eignet sich zur Durchführung von Hydrierungen in einem mehrphasigen System. Im einen solchen mehrphasigen System stellt dabei Wasserstoff die Gasphase dar, während die organische Verbindung in der flüssigen Phase vorliegt. In der flüssigen Phase kann ein Katalysator gelöst sein oder der Katalysator kann als feste Phase in Suspension in der flüssigen Phase vorliegen.

In das erfindungsgemäße Verfahren wird Wasserstoff eingesetzt.
Der zur Hydrierung verwendete Wasserstoff wird im Allgemeinen im größeren stöchiometrischen Überschuss vom 1- bis 10-fachen, bevorzugt vom 1,1- bis 5-fachen der stöchiometrisch notwendigen Mengen verwendet. Er kann als Kreisgas in die Reaktion zurückgeführt werden. Der Wasserstoff kommt im Allgemeinen technisch rein zum Einsatz. Der Wasserstoff kann auch in Form eines Wasserstoff enthaltenden Gases, d.h. in Beimengungen mit anderen Inertgasen, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden. Bevorzugt wird jedoch reiner Wasserstoff bzw. im Wesentlichen reiner Wasserstoff in das Verfahren eingesetzt.

In das erfindungsgemäße Verfahren werden weiterhin organischen Verbindungen, die mindestens eine ungesättigte Kohlenstoff-Kohlenstoff-, Kohlenstoff-Stickstoff- oder Kohlenstoff-Sauerstoff-Bindungen enthalten, eingesetzt.
Beispiele für solche organischen Verbindungen, sind Verbindungen, die mindestens eine oder mehrere Carbonsäureamidgruppen, Nitrilgruppen, Imingruppen, Enamingruppen, Azidgruppen oder Oximgruppen enthalten, die zu Aminen hydriert werden.

Weiterhin können in dem erfindungsgemäßen Verfahren Verbindungen, die mindestens eine oder mehrere Carbonsäureestergruppen, Carbonsäuregruppen, Aldehydgruppen oder Ketogruppen enthalten, die zu Alkoholen hydriert werden.
In das Verfahren können auch organische Verbindungen mit ungesättigten Kohlenstoff-Kohlenstoff-Bindungen, wie Alkene und/oder Alkine, eingesetzt werden.
Als organische Verbindungen kommen auch Aromaten oder Heteroaromaten in Betracht, die partiell oder vollständig zu ungesättigten oder gesättigten Carbo- oder Heterozyklen umgesetzt werden können.
Vorzugsweise werden als organische Verbindungen aromatische Verbindungen eingesetzt.
Als aromatische Ausgangsstoffe für die Hydrierung nach dem erfindungsgemäßen Verfahren kommen in Betracht:
aromatische Monoamine, wie Anilin, die isomeren Toluidine, die isomeren Xylidine, 1- bzw. 2-Aminonaphthalin, Benzidin und substituierte Benzidine;
aromatische Diamine, wie die isomeren Phenylendiamine (o-Phenylendiamin, m-Phenylendiamin, p-Phenylendiamin), die isomeren Tolylendiamine, wie 2,4-Diaminotoluol und/oder 2,6 Diaminotoluol, die isomeren Diaminonaphthalene, wie 1,5-Diaminonaphthalin, Bis-(4-aminophenyl)-methan (MDA), Meta-Xylendiamin (MXDA), Bis-(4-amino-3-methylphenyl)-methan und Bis-(4-amino-3,5-dimethylphenyl)-methan;
aromatische Polyamine, wie Polymer-MDA (Polymethylen-Polyphenyl-Amin);
aromatische Mono- und Dicarbonsäuren und deren Ester, wie Benzoesäure, die isomeren Phthalsäuren sowie deren Ester, bevorzugt deren Methylester,
aromatische Aminocarbonsäuren und deren Ester, wie Anthranilsäure;
aromatische Alkohole, wie Phenol und Bisphenol A; sowie
aromatische Kohlenwasserstoffe, wie Benzol, Toluol, Ethylbenzol, die isomeren Xylole, Inden, Tetralin und Naphthalin.

In das Verfahren können auch substituierte aromatische Verbindungen eingesetzt werden, bei denen neben der Hydrierung des aromatischen Kerns auch eine Hydrierungder Substituenten eintreten kann. Beispiele hierfür sind aromatische Monoamine, die Nitrogruppen aufweisen, wie die isomeren Nitroaniline, oder aromatische Ketone, wie Acetophenon, oder substituierte Nitrile, wie Benzonitril, Toluonitril bzw. o-Aminobenzonitril.

Zudem können aromatische stickstoffhaltige Heterocyclen, wie Pyridin, Pyrrol oder Indol in das erfindungsgemäße Verfahren eingesetzt werden.

Bevorzugt werden in das Verfahren aromatische Amine, wie die voranstehend genannten aromatischen Mono-, Di- und/oder Polyamine, eingesetzt.

Besonders bevorzugt wird Polymer-MDA, Anilin, 2,4-Diaminotoluol, 2,6-Diaminotoluol, o-Phenylendiamin, m-Phenylendiamin, p-Phenylendiamin, Bis-(4-aminophenyl)-methan (MDA), Meta-Xylendiamin (MXDA), Bis-(4-amino-3-methylphenyl)-methan und/oder Bis-(4-amino-3,5-dimethylphenyl)-methan in das Verfahren eingesetzt.
Ganz besonders bevorzugt werden Anilin, Meta-Xylendiamin (MXDA), Bis-(4-aminophenyl)-methan (MDA), Bis-(4-amino-3-methylphenyl)-methan und Bis-(4-amino-3,5-dimethylphenyl)-methan in das Verfahren eingesetzt.

Die aromatischen Amine können zusätzlich zu den Aminogruppen keine weiteren Substituenten aufweisen oder sie können ein oder mehrere weitere Substituenten tragen, beispielsweise Alkyl-, Cycloalkyl-, Aryl-, Heteroaryl-, Halogen-, Halogenalkyl-, Silyl-, Hydroxy-, Alkoxy-, Aryloxy-, Carboxy- oder Alkoxycarbonyl-Substituenten.

Die durch das erfindungsgemäße Verfahren erhältlichen cycloaliphatischen Amine können als Synthesebaustein für die Herstellung von Tensiden, Arznei- und Pflanzenschutzmitteln, Stabilisatoren, Lichtschutzmitteln, Polymeren, Polyamiden, Isocyanaten, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, lonenaustauschern, Textilhilfsmitteln, Farbstoffen, Vulkanisationsbeschleunigern, Emulgatoren und/oder als Ausgangssubstanzen für die Herstellung von Harnstoffen und Polyharnstoffen verwendet werden.
Insbesondere kann Cyclohexylamin, erhältlich durch die Hydrierung von Anilin, als Korrosionsinhibitor oder Vulkanisationsbeschleuniger verwendet werden.
Die Hydrierprodukte von Bis-(4-aminophenyl)-methan (MDA), Bis-(4-amino-3-methylphenyl)-methan und/oder Bis-(4-amino-3,5-dimethylphenyl)-methan können als Monomerbaustein für Polyamide, als Härter für Epoxyharze oder als Ausgangsprodukt für die Herstellung der entsprechenden Isocyanate verwendet werden.
Die Hydrierung kann mit oder ohne Lösungsmittel durchgeführt werden. Der Einsatz von Lösungsmitteln ist vorteilhaft, wenn die organische Verbindung als Feststoff vorliegt und sich nicht oder nur unter großem Aufwand als Schmelze handhaben und fördern lässt. Als Lösungsmittel können Alkohole, wie Isopropanol, Isobutanol oder t-Butanol, oder Ether, wie Diethylether, Glykoldimethylether, Dioxan oder Tetrahydrofuran, verwendet werden.
Als Lösungsmittel kann aber auch das bei der Reaktion entstehende Endprodukt eingesetzt werden.

Als Lösungsmittel kommen auch Mischungen der voranstehend genannten Lösungsmittel in Betracht.
Bevorzugte Lösungsmittel sind Isopropanol, Isobutanol und/oder t-Butanol. Besonders bevorzugt wird als Lösungsmittel das bei der Reaktion entstehende Endprodukt eingesetzt.
Das Lösungsmittel wird gewöhnlich in einer solchen Menge eingesetzt, so dass man 10 bis 50%ige (Gew.-%), bevorzugt 15 bis 40%ige, besonders bevorzugt 20 bis 30%ige Lösungen der zur Hydrierung vorgesehenen organischen Verbindungen erhält. Besonders bevorzugt wird das Verfahren ohne Verwendung eines Lösungsmittels durchgeführt.

Das erfindungsgemäße Verfahren erfolgt in Gegenwart eines homogenen oder heterogenen Katalysators, der für Hydrierungen geeignet ist. Vorzugsweise wird die Hydrierung in Gegenwart eines heterogenen Katalysators durchgeführt.
Als homogene Katalysatoren kommen flüssige und/oder lösliche Hydrierkatalysatoren in Betracht, beispielsweise Wilkinson-Katalysatoren, Crabtree-Katalysatoren oder Lindlar-Katalysatoren.
Als heterogene Katalysatoren, werden beispielsweise Edelmetalle wie Platin, Palladium, Ruthenium und Rhodium oder andere Übergangsmetalle wie Molybdän, Wolfram, Chrom, besonders aber Eisen, Cobalt und Nickel verwendet, entweder einzeln oder im Gemisch.
Zur Erhöhung der Aktivität und Stabilität können heterogene Katalysatoren in fein verteilter Form in das Verfahren eingesetzt werden.
Die Katalysatoren können Trägermaterial enthalten.
Als Trägermaterial werden üblicherweise Kohlenstoff, wie Graphit, Russ und/oder Aktivkohle, Aluminiumoxid, Siliziumdioxid, Zirkoniumdioxid, Zeolithe und Alumosilicate sowie Mischungen aus diesen Trägermaterialien verwendet.
Hierbei kann die Form und Größe des Trägermaterials variieren. Typische mittlere Korngrößen von Trägermaterialien liegen im Bereich von 0,0001 bis zu 5 Millimetern, vorzugsweise 0,0005 bis 1 mm, besonders bevorzugt 0,001 bis 0,5 mm.
Die Konzentration des Katalysators beträgt üblicherweise 1 Gew.-% und weniger, bevorzugt 0,1 Gew.-% und weniger, und besonders bevorzugt 0,01 Gew.-% und weniger bezogen auf die Summe bestehend aus der Masse der eingesetzten organischer Verbindung und der Masse des eingesetzten Lösungsmittels.

In einer besonderen Ausführungsform wird als heterogener Ru-haltiger Katalysator bevorzugt ein Katalysator auf Basis von Ru-Oxidhydrat zur Hydrierung von aromatischen Verbindungen eingesetzt. Besonders bevorzugt wird ein Ru-haltiger Katalysator eingesetzt, wie er in der DE-A1-2132547 offenbart ist. Die Herstellung solcher bevorzugter Katalysatoren ist beispielsweise auf den Seiten 4 bis 5 sowie in dem Beispiel 1 der DE-A1-2132547 näher beschrieben. Typischerweise liegt die mittlere Teilchengröße von heterogenen Ru-haltigen Katalysatoren, die durch Fällung erhalten wurden, im Bereich von 1 nm bis 1 µm, bevorzugt im Bereich von 3 nm bis 100 nm. Beispielsweise beträgt die mittlere Teilchengröße der Rutheniumpartikel, die als Oxidhydrate des Ru eingesetzt werden, gemäß der Offenbarung von DE-A1-2132547 zwischen 4 und 6 nm.

Die heterogenen Katalysatoren werden üblicherweise als Suspension des Katalysators in den eingesetzten flüssigen Edukten bzw. Lösungsmitteln eingesetzt.
Das erfindungsgemäße Verfahren wird üblicherweise bei einem Druck von 1 bis 500 bar durchgeführt, vorzugsweise bei einem Druck von 50 bis 325 bar, besonders bevorzugt einem Druck von 150 bis 250 bar.
Das Verfahren wird in der Regel bei einer Temperatur im Bereich von 50 und 300°C durchgeführt, wobei der Temperaturbereich von 120 bis 280°C bevorzugt wird.

Das erfindungsgemäße Verfahren wird in zwei Stufen durchgeführt.

Die erste Stufe wird in einem Schlaufenreaktor mit externem Wärmetauscher durchgeführt.

Beispiele für Schlaufenreaktoren sind Rohrreaktoren mit internen und externen Schlaufen. Solche Reaktoren sind beispielsweise in Ullmann's Encyclopädie näher beschrieben (Ullmann's Encyclopedia of Industrial Chemistry, Verlag Chemie, Electronic Release 2008, 7th Edition, Kapitel "Stirred Tank and Loop Reactors" und Kapitel "Bubble Columns").

Ein Schlaufenreaktor besteht üblicherweise aus einem vertikalen, vorzugsweise zylindrischen Rohrreaktor.
Das Verhältnis von Länge zu Durchmesser des Schlaufenreaktors beträgt üblicherweise 2:1 bis 100:1, bevorzugt 5:1 bis 100:1, besonders bevorzugt 5:1 bis 50:1, insbesondere bevorzugt 5:1 bis 30:1.
Dem Rohrreaktor werden die Edukte, einzeln oder gemischt, zugeführt.
Die Zuführung der Edukte erfolgt vorzugsweise im unteren Bereich des Rohrreaktors, vorzugsweise durch eine im unteren Bereich des Schlaufenreaktors angeordneten Mischdüse.
Der Begriff Mischdüse bezeichnet in üblicher Weise ein sich in Strömungsrichtung verjüngendes Rohr.
Wasserstoffgas, Edukte, die ggf. in einem Lösungsmittel gelöst sind sowie Katalysatorlösung bzw. Katalysatorsuspension werden im Allgemeinen intensiv in der Mischdüse vermischt und dem Reaktor zugeführt.
Die Mischdüse kann als Einstoff- oder Zweistoffdüse ausgelegt werden.
Bei der Einstoffdüse wird nur das flüssige Reaktionsgemisch eingedüst und der Wasserstoff an einer beliebig anderen Stelle, vorzugsweise jedoch in der Flüssigphase, dem Reaktor zugeführt, beispielsweise durch einen Begasungs- oder Verteilerring.

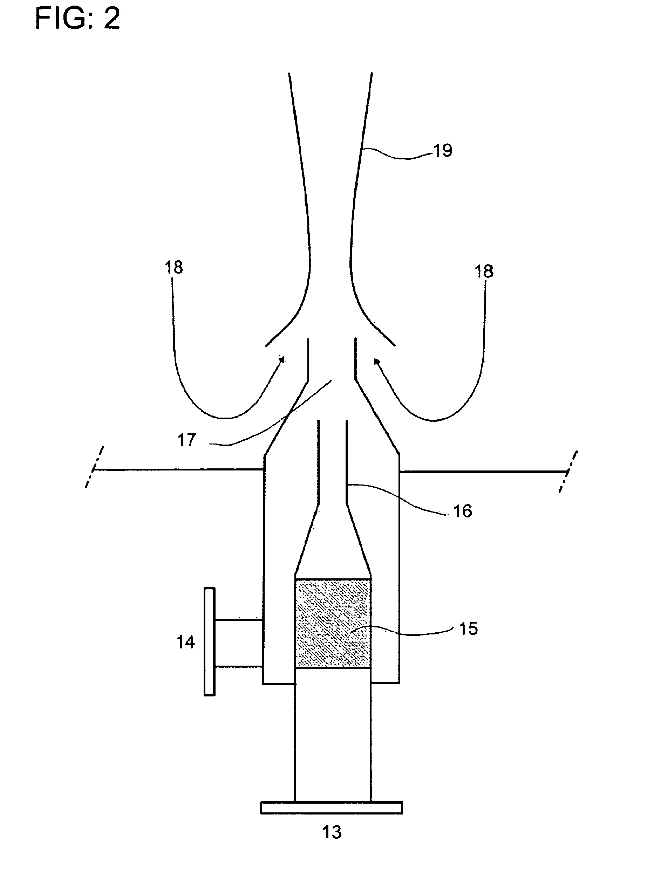

Vorteilhaft bei dieser Ausgestaltung ist der einfache Aufbau einer solchen Einstoffdüse, nachteilig ist die schlechtere Dispergierung des Wasserstoffs im Reaktionsgemisch. Bei der Zweistoffdüse wird der Wasserstoff zusammen mit dem flüssigen Reaktionsgemisch zugeführt und dispergiert. Bei dieser Ausführungsform wird üblicherweise eine hohe Dispergierung des Wasserstoffs in der Reaktionsmischung erzielt.
Bevorzugt ist die Mischdüse als Zweistoffdüse ausgestaltet.
Im Rahmen der vorliegenden Anmeldung wird als Zweistoffdüse auch eine Düse bezeichnet, bei dem sich im flüssigen Reaktionsgemisch suspendierte Katalysatorpartikel befinden.
Eine bevorzugte Ausführungsform der Mischdüse ist in Figur 2 abgebildet und wird untenstehend näher beschrieben.

Der Schlaufenreaktor ist im Allgemeinen als Rohrreaktor mit externem Kreislauf (externe Schlaufen) gestaltet.
Bei einem Schlaufenreaktor mit externem Kreislauf befindet sich in der Regel ein Abzug an beliebiger Stelle des Reaktors, vorzugsweise im unteren Bereich des Reaktors, über den das Reaktionsgemisch in einem äußeren Kreislauf mittels eines Förderorgans der Mischdüse wieder zugeführt wird.
Das Förderorgan ist vorzugsweise eine Pumpe, weshalb der externe Kreislauf üblicherweise als Umpumpkreislauf bezeichnet wird.
Beispiele für Pumpen sind Kreiselpumpen oder Rotationskolbenpumpen, wie Drehkolbenpumpen, Drehschieberpumpen, Kreiskolbenpumpen oder Zahnradpumpen. Besonders bevorzugt werden Kreiselpumpen als Förderorgan verwendet.

Im erfindungsgemäßen Verfahren wird die erste Stufe in einem Schlaufenreaktor mit externen Kreislauf durchgeführt, wobei sich in im externen Kreislauf ein Wärmeaustauscher befindet. Ein solcher Reaktor wird im Rahmen dieser Erfindung als Schlaufenreaktor mit externem Wärmetauscher bezeichnet.

Der Wärmetauscher ist beispielsweise ein Rohrbündelwärmetauscher, Doppelrohrwärmetauscher, Plattenwärmetauscher oder Spiralwärmetauscher. Bei Auslegungsdrücken für den Reaktor unter 100 bar wird bevorzugt ein Rohrbündelwärmetauscher verwendet, bei höheren Drücken wird bevorzugt ein oder mehrere hintereinander geschaltete Doppelrohrwärmetauscher verwendet.

Der Schlaufenreaktor mit externem Wärmetauscher wird dabei üblicherweise so betrieben, dass ein Teil des Reaktionsgemisches aus dem Reaktor durch den externen Umpumpkreislauf, in dem sich der externe Wärmetauscher befindet, gefördert wird, wobei das durch den Wärmetauscher geförderte Reaktionsgemisch gekühlt wird. Durch das externe Umpumpen wird im Allgemeinen eine so genannte externe Schlaufenströmung erzeugt. Dadurch wird das Reaktionsgemisch in der ersten Reaktionsstufe in der Regel so stark durchmischt und umgewälzt, so dass die Verweilzeit in der ersten Stufe üblicherweise der eines vollständig rückvermischten Rührkessels (CSTR) entspricht.
Das Reaktionsgemisch wird schließlich mittels Mischdüse wieder dem Reaktor zugeführt.
Üblicherweise werden frische Edukte, Wasserstoffgas und Katalysatorlösung bzw. Katalysatorsuspension in den Umpumpkreislauf dosiert und zusammen mit dem bereits im Umpumpkreislauf befindlichen Strom als Reaktionsgemisch dem Reaktor zugeführt, bevorzugt mittels Mischdüse. In die Mischdüse wird in der Regel auch Wasserstoffgas zugeführt, das vom Reaktionsgemisch in einem Gasabscheider nach der ersten und/oder zweiten Reaktionsstufe abgetrennt wird (Kreisgas).

In einer bevorzugten Ausführungsform ist der Schlaufenreaktor so gestaltet, dass zusätzlich zur externen Schlaufenströmung eine so genannte interne Schlaufenströmung ausgebildet wird. In einem Schlaufenreaktor mit interner Schlaufenströmung ist in der Regel im Inneren des Rohrreaktors ein konzentrisches, vorzugsweise zylindrisches Leitrohr angeordnet, das sich im Wesentlichen über die gesamte Länge des Rohrreaktors mit Ausnahmen der Reaktorenden erstreckt.
Das Leitrohr ist in der Regel als einfaches Rohr ausgestaltet. Das Verhältnis von Länge zu Durchmesser des Leitrohrs beträgt im Allgemeinen 5:1 bis 100:1, bevorzugt 5:1 bis 50:1.
Der Durchmesser des Leitrohrs ist üblicherweise geringer, als der Durchmesser des Rohrreaktors. Das Verhältnis des Durchmessers des Leitrohrs zum Durchmesser des Rohrreaktors beträgt in der Regel 0,3:1 bis 0,9:1, bevorzugt 0,5:1 bis 0,7:1.
Der Raum zwischen Leitrohr und der Reaktorinnenwand wird im Allgemeinen als Ringraum bezeichnet.
Die Mischdüse ist üblicherweise so angeordnet, dass der von der Mischdüse erzeugte Gas/Flüssigkeitsstrahl in das Leitrohr gerichtet ist.
Die Mischdüse ist bevorzugt unterhalb des unteren Ende des Leitrohrs, insbesondere um 1/8 des Leitrohrdurchmessers bis zu einem Leitrohrdurchmesser beabstandet angeordnet oder taucht in das Leitrohr, in eine Tiefe bis zu mehreren Leitrohrdurchmessern ein.
Der mittels Mischdüse erzeugte Gas/Flüssigkeitsstrahl bewirkt eine Strömung im Leitrohr (Aufstromsäule), die nach Verlassen des Leitrohrs umgelenkt wird, so dass die Flüssigkeit im Ringraum zwischen Leitrohr und Reaktorinnenwand in Richtung der Mischdüse zurückgeführt wird (Abstromsäule). So entsteht in der Regel eine interne Schlaufenströmung. Befindet sich die Mischdüse am unteren Ende des Rohrreaktors, so wird üblicherweise die interne Schlaufenströmung durch die sich im Leitrohr bildende Blasensäule durch Ausnutzung der Gasauftriebskraft verstärkt.
Das Verhältnis der Volumenströme von interner Schlaufenströmung zu extern umgepumpten Reaktionsgemisch beträgt vorzugsweise 2 bis 30:1, besonders bevorzugt 5 bis 20:1.

Der Schlaufenreaktor mit interner Schlaufenströmung kann auch mit zwei getrennten Rohrleitungen ausgeführt werden, wobei in einer ersten Rohrleitung die Aufwärtsströmung durchgeführt wird, oberhalb von dieser Rohrleitung Gas und Flüssigkeit getrennt werden und die Flüssigkeit in einer zweiten Rohrleitung wieder nach unten zur Mischdüse geführt wird.

Der Schlaufenreaktor mit externem Umpumpkreislauf mit Wärmetauscher kann auch als Rührkesselreaktor mit externem Umpumpkreislauf mit Wärmetauscher ausgeführt sein. In dieser Ausführungsform kann die Zuführung der Edukte, wie voranstehend beschrieben, vorzugsweise durch Zuführung in einen Umpumpkreislauf und Einleiten des Reaktionsgemischs mittels einer Mischdüse, erfolgen. Bevorzugt erfolgt die Zuführung der Edukte in den unteren Teil des Reaktors. Durch das Rühren wird in der Regel eine gute Mischung im Reaktor erzielt, und die Reaktionswärme kann über den externen Wärmetauscher abgeführt werden.

Bevorzugt wird die erste Verfahrensstufe jedoch in einem Schlaufenreaktor mit interner Schlaufenströmung durchgeführt.

Aus der ersten Reaktionsstufe wird üblicherweise ein Teil der Reaktionsmischung der zweiten Reaktionsstufe zugeführt.

Die zweite Reaktionsstufe ist erfindungsgemäß ein Blasensäulenreaktor mit begrenzter Rückmischung, d.h. dass die Zirkulation von Gas und Flüssigkeit begrenzt wird, so dass die Verweilzeitverteilung in dieser zweiten Reaktionsstufe der eines Rohrreaktors entspricht.
Durch diese definierte Flüssigkeitsverweilzeit kann der Umsatz der Edukte nahezu vollständig erfolgen. Zudem werden in der zweiten Reaktionsstufe in der Regel keine weiteren Kühlvorrichtungen benötigt, da nur noch geringe Reaktionswärme freigesetzt wird.

Der Blasensäulenreaktor besteht üblicherweise aus einem vertikalen, vorzugsweise zylindrischen Rohrreaktor.
Das Verhältnis von Länge zu Durchmesser des Blasensäulenreaktors beträgt üblicherweise 2:1 bis 100:1, bevorzugt 5:1 bis 50:1, besonders bevorzugt 10:1 bis 30:1.

Die Rückmischung im Blasensäulenreaktor der zweiten Verfahrensstufe wird vorzugsweise durch Einbauten begrenzt. Durch den Einbau solcher Vorrichtungen werden in der Regel die Zirkulation und damit die Rückvermischung von Gas- und Flüssigkeit eingeschränkt. Die Einbauten sind üblicherweise so beschaffen, dass die mehrphasige Mischung bestehend aus Gas, Flüssigkeit und gelöstem bzw. suspendiertem Katalysator gemeinsam hindurchströmen kann, ohne dass es zu einer Entmischung der Phasen oder auch zur Sedimentation und Ablagerung von Feststoff in diesem Reaktionsteil kommt.

Die Begrenzung der Rückmischung im Blasensäulenreaktor kann beispielsweise durch den Einbau verschiedener Einbauten realisiert werden:

In einer bevorzugten Ausdührungsform erfolgt die Begrenzung der Rückmischung durch den Einbau von mehreren, fest angeordneten Böden in den Rohrreaktor.
Im Allgemeinen entstehen zwischen den einzelnen Böden somit Einzelsegmente ("Kompartments") mit definierten Reaktionsvolumina. Jedes der Einzelsegmente wirkt in der Regel dabei wie ein einzelner, rückvermischter Rührkesselreaktor. Mit zunehmender Anzahl von Einzelsegmenten hintereinander nähert sich die Verweilzeitverteilung einer derartigen Kaskade in der Regel der Verweilzeit eines Rohrreaktors an. Die Böden sind dabei bevorzugt als flüssigkeitsdurchlässige Begasungsböden ausgeführt. Beispiele für solche Begasungsböden sind einfache Lochbleche und Schlitzböden, wobei die Bleche bzw. Böden mit Begasungsdüsen oder mit Zweistoff-Ventilen versehen sein können, und speziell konstruierte Begasungsböden, wie beispielsweise Tüllenböden oder Thormannböden, Tunnelböden oder Kreuzstromböden.
Vorzugsweise sind die Begasungsböden als Lochbleche, Schlitzböden, Tüllenböden, Thormannböden, Tunnelböden oder Kreuzstromboden, ausgeführt. Besonders bevorzugt sind die Begasungsböden als Lochbleche gestaltet.
Das Volumen des Blasensäulenreaktors wird üblicherweise durch die eingebauten Böden in etwa gleichgroße Einzelsegmente unterteilt.
Die Anzahl der so gebildeten Einzelsegmente beträgt vorzugsweise 1 bis 20, besonders bevorzugt 1 bis 10, insbesondere bevorzugt 2 bis 6.
Das Volumen der Einzelsegmente beträgt bevorzugt jeweils 5 bis 50%, besonders bevorzugt 10 bis 25%, bezogen auf das Gesamtvolumen des Blasensäulenreaktors. Es ist jedoch auch, je nach Reaktionssystem und freigesetzter Reaktionswärme, eine andere Verteilung der einzelnen Segmente möglich.

In einer weiteren bevorzugten Ausführungsform erfolgt die Begrenzung der Rückmischung durch den Einbau von unregelmäßig aufgeschütteten Füllkörpern oder durch den Einbau einer strukturierten Packung im gesamten Volumen des Blasensäulenreaktors.
Bei Füllkörpern und strukturierten Packungen wird dabei bevorzugt eine Bauform gewählt, welche eine weitere Vermischung von Flüssigkeit- und Gasphase bewirkt und gleichzeitig einen geringen Flüssigkeits-Hold-Up besitzt um die Sedimentation von Katalysator zu verhindern Das verwendete Material sollte in der Regel deshalb möglichst unporös sein und gleichzeitig keine strömungsberuhigten Zonen aufweisen. Bevorzugt sollte die Oberfläche zur Vermeidung von Feststoffablagerungen möglichst glatt sein, wie beispielsweise bei Metall oder glasierter Keramik.

Als Füllkörper können beispielsweise Pall®Ringe oder Raschig®-Ringe eingesetzt werden.
Als Bauform der strukturierten Packungen können in der Regel alle Bauformen verwendet werden, welche ansonsten üblicherweise als Einbauten in Destillationskolonnen zum Einsatz kommen, beispielsweise die Typen Mellapack®, Optiflow®, Mellagrid®, BX®, CY®, Mellacarbon®, Melladur®, Kerapak® (Fa. Sulzer Chemtech), die Typen Montz-Pak® der Fa. Montz sowie Flexipac® (Fa. Koch-Glitsch).

Der Schlaufenreaktor der ersten Reaktionsstufe und der Blasensäulenreaktor der zweiten Reaktionsstufe können als zwei räumlich voneinander getrennte Apparate angeordnet sein, die beispielsweise über Rohrleitungen miteinander verbunden sind.

In einer bevorzugten Ausführungsform sind jedoch beide Reaktionsstufen, nämlich Schlaufenreaktor und Blasensäulenreaktor, in einem Apparat (Hydrierreaktor) angeordnet.
In dieser bevorzugten Ausführungsform ist der Hydrierreaktor als langes, zylindrisches Rohr ausgeführt (hochzylindrische Bauform).
Das Verhältnis von Gesamtlänge zu Durchmesser des Rohrs liegt bevorzugt im Bereich von 5:1 bis 100:1, besonders bevorzugt 5:1 bis 50:1, ganz besonders bevorzugt 10:1 bis 30:1.
Das zylindrische Rohr wird üblicherweise in zwei Bereiche unterteilt.
Der erste Bereich des Hydrierreaktors ist wie voranstehend beschrieben als Schlaufenreaktor mit externem Wärmetauscher ausgestaltet.
Der zweite Bereich des Hydrierreaktors ist wie voranstehend beschrieben als Blasensäulenreaktor ausgestaltet.
Der Bereich des Schlaufenreaktors und der des Blasensäulenreaktors werden in der Regel durch Einbauten, vorzugsweise Begasungsböden, wie Lochblenden, voneinander getrennt.
Der Bereich des Blasensäulenreaktors beginnt üblicherweise ab dem ersten Einbauelement, das die Rückvermischung begrenzt. Hierbei wird das erste Einbauelement bereits zum Bereich des Blasensäulenreaktors gezählt.
Der Bereich bis zum ersten Einbauelement, das die Rückvermischung begrenzt, wird dem Bereich des Schlaufenreaktors zugeordnet.
Das Verhältnis des Volumens des oberen Bereichs des Reaktors, in dem die Einbauten angebracht sind und in dem die zweite Stufe des Hydrierverfahrens durchgeführt wird, zum Volumens des unteren Bereichs des Reaktors, in dem die erste Stufe des Verfahrens durchgeführt wird, liegt bevorzugt im Bereich von 0,1:1 bis 10:1, besonders bevorzugt im Bereich von 0,3:1 bis 2:1, ganz besonders bevorzugt im Bereich von 0,4:1 bis 1,5:1, insbesondere 1:1.

Das Volumen des oberen Bereichs des Reaktors, in dem die zweite Verfahrensstufe durchgeführt wird, wird durch die Begasungsböden in Einzelsegmente (Kompartments) unterteilt, wobei bevorzugt das Volumen eines durch die Begasungsböden unterteilten Einzelsegments im Bereich von 5 bis 50%, bezogen auf das Gesamtvolumen des Reaktors, beträgt.
Die Anzahl der Einzelsegmente beträgtvorzugsweise1 bis 20, besonders bevorzugt 1 bis 10, insbesondere 2 bis 6.

In einer ganz besonders bevorzugten Ausführungsform liegt das Verhältnis des Volumens des oberen Bereichs des Reaktors, in dem die Einbauten angebracht sind und in dem die zweite Stufe des Hydrierverfahrens durchgeführt wird, zum Volumens des unteren Bereichs des Reaktors, in dem die erste Stufe des Verfahrens durchgeführt wird, im Bereich von 0,4:1 bis 1,5:1, insbesondere 1:1, bei einer Anzahl von 2 bis 6 Einzelsegmenten. Diese bevorzugte Ausführungsform ermöglicht eine ausreichende Verweilzeit zum Erzielen von hohen Umsätzen, auch bei Umsatz- und Lastschwankungen in der ersten Reaktionsstufe.

Das erfindungsgemäße Verfahren wird in der bevorzugten Ausführungsart, in der Schlaufenreaktor und Blasensäulenreaktor in einem Apparat integriert sind, vorzugsweise derart betrieben, dass zwischen der Eduktzuführung im Schlaufenreaktors der ersten Reaktionsstufe und dem Austritt des Gas-Flüssigkeitsgemisches im Blasensäulenreaktor der zweiten Reaktionsstufe eine maximale Temperaturdifferenz von 20 K, bevorzugt kleiner als 10 K, auftritt.
Die Temperatur im Schlaufenreaktor kann dabei sehr genau durch die Kreislaufmenge und die Dimensionierung des Kühlers im Umpumpkreislauf festgelegt werden.
Die Regelung des Umsatzes in diesem Reaktor erfolgt dabei vorzugsweise über die Menge an zugeführtem Katalysator. Zur Erzielung eines möglichst vollständigen Umsatzes am Austritt des Blasensäulenreaktors und zur Begrenzung der maximal auftretenden Temperaturdifferenz über den gesamten Reaktor auf kleiner 20 K, bevorzugt kleiner 10 K, hat es sich als vorteilhaft herausgestellt, im Schlaufenreaktor der ersten Verfahrensstufe einen Umsatz im Bereich von 90 - 98%, bevorzugt 92 bis 98%, einzustellen.
Der Umsatz in der ersten Verfahrensstufe wird dabei vorzugsweise durch die Konzentration der zugeführten Katalysatorlösung bzw. Katalysatorsuspension reguliert. Dabei kann in der Regel durch eine Erhöhung der Katalysatorkonzentration der Umsatz in der ersten Verfahrensstufe erhöht werden. Die Kontrolle des Umsatzes kann durch Probenahme im Umpumpkreislauf oder besonders bevorzugt über geeignete On-Line Analysenmethoden, vorzugsweise durch Infrarot- oder Nah-Infrarot-Messungen, erfolgen.
Die zweite Reaktionsstufe ist im Allgemeinen so angeordnet, so dass das Reaktionsgemisch mittels der durch den Gasauftrieb erzeugten Strömung aus dem Bereich, der als Schlaufenreaktor ausgeführt ist, in die Einbauten des Blasensäulenreaktors gelangt.

Am Kopf der zweiten Reaktionsstufe (Blasensäulenreaktor) treten Flüssigkeit und Gas gemeinsam aus dem Reaktor (Hydrierreaktor) aus und werden im Allgemeinen in einem nachgeschalteten Abscheidebehälter (Gasabscheider) voneinander getrennt. Das abgeschiedene Gas wird üblicherweise der Mischdüse in der ersten Reaktionsstufe wieder zugeführt (Kreisgas).

Der Schlaufenreaktor der ersten Reaktionsstufe und der Blasensäulenreaktor der zweiten Reaktionsstufe können wie voranstehend erwähnt auch als zwei getrennte Apparate angeordnet sein.
In einer solchen alternativen Ausführungsform ist der Schlaufenreaktor, wie zuvor beschrieben, als Schlaufenreaktor mit externem Wärmetauscher ausgeführt.
Der Blasensäulenreaktor ist wie voranstehend beschrieben als Rohrreaktor mit Einbauten, die die Rückmischung begrenzen, gestaltet.
In einem Schlaufenreaktor mit internen Schlaufen kann im oberen Bereich des Schlaufenreaktors oberhalb des oberen Endes des Leitrohres eine Prallplatte angeordnet sein. Die Prallplatte ist in der Regel senkrecht zum Leitrohr angeordnet und von diesem bevorzugt um einen bis zwei Leitrohrdurchmesser beabstandet. Die Prallplatte ist bevorzugt scheibenförmig ausgebildet, mit einem Durchmesser größer als der Durchmesser des Leitrohrs und kleiner als der Innendurchmesser des Schlaufenreaktors. Die Dicke beträgt im Allgemeinen etwa 5 bis 10 mm. Die Prallplatte hat üblicherweise die Aufgabe eine Strömungsumkehr im oberen Bereich des Reaktors zu bewirken.

In dieser alternativen Verfahrensvariante, in der die Reaktionsstufen als zwei getrennte Apparate ausgeführt sind, werden üblicherweise Wasserstoffgas und das flüssige Reaktionsgemisch in den Schlaufenreaktor, bevorzugt mittels einer Mischdüse, zugeführt. An einer beliebigen Stelle des Schlaufenreaktors, beispielsweise im unteren Bereich oder im Umpumpkreislauf - allerdings vor der Dosierung der Edukte - wird ein Teil des flüssigen Reaktionsgemischs abgezweigt und der zweiten Reaktionsstufe zugeführt. Die Zuführung des Reaktionsgemisch aus der ersten Verfahrensstufe kann mittels einer Pumpe erfolgen, die Zuführung kann aber auch in derart erfolgen, dass die zweite Verfahrensstufe bei einem etwas geringeren Reaktionsdruck betrieben wird und Reaktionsgemisch durch die Druckdifferenz in die zweite Verfahrensstufe gefördert wird. Das Reaktionsgemisch aus der ersten Stufe wird üblicherweise in den Blasensäulenreaktor der zweiten Stufe, bevorzugt über eine Düse, bevorzugt zusammen mit Wasserstoffgas, zudosiert. Die Zufuhr von Wasserstoffgas kann aber auch getrennt, beispielsweise durch einen Gaseinleitungs- oder Verteilerring, vorzugsweise in den unteren Bereich des Blasensäulenreaktors, erfolgen. Im unteren Bereich des Blasensäulenreaktors, in dem die Zufuhr der Edukte, insbesondere Wasserstoffgas erfolgt, befinden sich üblicherweise keine Einbauten. Dieser Bereich, in dem sich üblicherweise keine Einbauten befinden, entspricht üblicherweise 5 bis 50% des Volumens des Blasensäulenreaktors der zweiten Reaktionsstufe. Werden als Einbauten Böden, wie Lochbleche verwendet, so kann die Einleitung von Wasserstoffgas in den Reaktionsraum vor dem ersten Boden bzw. in den Reaktionsraum zwischen erstem und zweitem Boden erfolgen.
Das Wasserstoffgas kann dabei frisch oder als Kreisgas zugeführt werden.
Am Kopf der zweiten Reaktionsstufe treten Flüssigkeit und Gas gemeinsam aus dem Blasensäulenreaktor aus und werden im Allgemeinen in einem nachgeschalteten Abscheidebehälter (Gasabscheider) voneinander getrennt. Das abgeschiedene Gas wird üblicherweise der Mischdüse in der ersten und/oder zweiten Reaktionsstufe wieder zugeführt (Kreisgas).
Am Kopf der ersten Reaktionsstufe wird ggf. Gas aus dem Reaktor abgeführt und im Allgemeinen in einem nachgeschalteten Abscheidebehälter (Gasabscheider) von Flüssigkeitsresten getrennt. Das abgeschiedene Wasserstoffgas wird üblicherweise der Mischdüse in der ersten und/oder zweiten Reaktionsstufe wieder zugeführt (Kreisgas). Die Reaktionsführung erfolgt dabei in Analogie zur Durchführung der bevorzugten Ausführungsform des Verfahrens, in dem Schlaufenreaktor und Blasensäulenreaktor in einem Apparat integriert sind, derart, dass zwischen der Eduktzuführung im Schlaufenreaktors der ersten Reaktionsstufe und dem Austritt des Gas-Flüssigkeitsgemisches im Blasensäulenreaktor der zweiten Reaktionsstufe eine maximale Temperaturdifferenz von 20 K, bevorzugt kleiner als 10 K auftritt. Die Regelung des Umsatzes erfolgt dabei vorzugsweise über die Menge an zugeführtem Katalysator. Zur Erzielung eines möglichst vollständigen Umsatzes am Austritt des Blasensäulenreaktors und zur Begrenzung der maximal auftretenden Temperaturdifferenz über den gesamten Reaktor auf kleiner 20 K, bevorzugt kleiner 10 K, hat es sich als vorteilhaft herausgestellt, im Schlaufenreaktor der ersten Verfahrensstufe einen Umsatz im Bereich von 90 - 98%, bevorzugt 92 bis 98%, einzustellen. Der Umsatz in der ersten Verfahrensstufe wird dabei vorzugsweise durch die Konzentration der zugeführten Katalysatorlösung bzw. Katalysatorsuspension reguliert. Dabei kann in der Regel durch eine Erhöhung der Katalysatorkonzentration der Umsatz in der ersten Verfahrensstufe erhöht werden.

Das erfindungsgemäße Verfahren wird jedoch bevorzugt in einem Reaktor durchgeführt, in dem die zwei Stufen des Verfahrens in einem Apparat angeordnet sind.

Geeignet ist ein Hydrierreaktor (1) in hochzylindrischer Bauform mit einem im unteren Reaktorbereich angeordneten konzentrischen Leitrohr (2) und einer nach oben gerichteten Mischdüse (3), über die Edukte und Reaktionsgemisch zugeführt werden können, einer Pumpe (4) sowie einem Wärmetauscher (5), die sich in einem externen Umpumpkreislauf befinden, der vom Reaktor zur Mischpumpe führt und mit ein oder mehreren im oberen Bereich des Reaktors angebrachten gas- und flüssigkeitsdurchlässige Einbauten (10), wobei das Verhältnis von Gesamtlänge zu Durchmesser des Hydrierreaktors (1) im Bereich von 5:1 bis 100:1 liegt und das Verhältnis des Volumens des oberen Bereich des Reaktors zum Volumen des unteren Bereichs des Reaktors 0,05:1 bis 10:1 beträgt.

Im unteren Bereich des Hydrierreaktor (1) wird die erste Stufe des erfindungsgemäßen Verfahrens ausgeführt, während im oberen Bereich des Hydrierreaktors (1) die zweite Stufe des Hydrierverfahrens durchgeführt wird.
Im unteren Bereich des Hydrierreaktors (1) ist im Inneren des Rohrreaktors ein konzentrisches, zylindrisches Leitrohr (2) angeordnet, das sich im Wesentlichen über die gesamte Länge des unteren Bereichs des Rohrreaktors bis zu den Einbauten des oberen Bereichs des Reaktors erstreckt, wobei jedoch die Einbauten um einen bis zwei Leitrohrdurchmesser vom oberen Ende des Leitrohres beabstandet sind.
Das Leitrohr (2) ist in der Regel als einfaches Rohr ausgestaltet. Das Verhältnis von Länge zu Durchmesser des Leitrohrs bevorzugt 5:1 bis 100:1, besonders bevorzugt 5:1 bis 25:1 und ganz besonders bevorzugt 10:1 bis 20:1.
Das Verhältnis des Durchmessers des Leitrohrs zum Durchmesser des Rohrreaktors beträgt bevorzugt 0,3:1 bis 0,9:1, besonders bevorzugt 0,5:1 bis 0,7:1.
Der Raum zwischen Leitrohr und der Reaktorinnenwand wird im Allgemeinen als Ringraum bezeichnet.
Die Mischdüse (3) ist üblicherweise so angeordnet, dass der von der Mischdüse erzeugte Gas/Flüssigkeitsstrahl in das Leitrohr gerichtet ist. Die Mischdüse ist bevorzugt unterhalb des unteren Ende des Leitrohrs, insbesondere um 1/8 des Leitrohrdurchmessers bis zu einem Leitrohrdurchmesser beabstandet angeordnet oder taucht in das Leitrohr, in eine Tiefe bis zu mehreren Leitrohrdurchmessern ein. Bevorzugt taucht das obere Ende der Mischdüse um einen Leitrohrdurchmesser in das Leitrohr ein.
Am Hydrierreaktor befindet sich ein Abzug, vorzugsweise im unteren Bereich des Reaktors, über den das Reaktionsgemisch in einem externen Umpumpkreislauf mittels einer Pumpe (4) der Mischdüse (3) wieder zugeführt wird.
Beispiele für Pumpen (4) sind Kreiselpumpen oder Rotationskolbenpumpen, wie Drehkolbenpumpen, Drehschieberpumpen, Kreiskolbenpumpen oder Zahnradpumpen. Besonders bevorzugt werden Kreiselpumpen als Förderorgan verwendet.

Der Begriff Mischdüse bezeichnet in üblicher Weise ein sich in Strömungsrichtung verjüngendes Rohr. Wasserstoffgas, Edukte, die ggf. in einem Lösungsmittel gelöst sind sowie Katalysatorlösung bzw. Katalysatorsuspension werden intensiv in der Mischdüse vermischt und dem Reaktor zugeführt.
Die Mischdüse kann als Einstoff- oder Zweistoffdüse ausgelegt werden.
Bei der Einstoffdüse wird nur das flüssige Reaktionsgemisch eingedüst und der Wasserstoff an einer beliebig anderen Stelle, vorzugsweise jedoch in der Flüssigphase, dem Reaktor zugeführt, beispielsweise durch einen Begasungs- oder Verteilerring. Vorteilhaft bei dieser Ausgestaltung ist der einfache Aufbau einer solchen Einstoffdüse, nachteilig ist die schlechtere Dispergierung des Wasserstoffs im Reaktionsgemisch. Bei der Zweistoffdüse wird der Wasserstoff zusammen mit dem flüssigen Reaktionsgemisch zugeführt und dispergiert. Bei dieser Ausführungsform wird üblicherweise eine hohe Dispergierung des Wasserstoffs in der Reaktionsmischung erzielt.
Bevorzugt ist die Mischdüse als Zweistoffdüse ausgestaltet.

Im Rahmen der vorliegenden Anmeldung wird als Zweistoffdüse auch eine Düse bezeichnet, bei dem sich im flüssigen Reaktionsgemisch suspendierte Katalysatorpartikel befinden. Eine bevorzugte Ausführungsform einer Mischdüse wird in Figur 2 abgebildet und ist untenstehend näher beschrieben.

In dem äußeren Kreislauf befindet sich ein Wärmetauscher (5).
Vorzugsweise befinden sich im äußeren Kreislauf Zuführungen (6, 7 und 8), die für die Einspeisung der Edukte vorgesehen sind.
Der Wärmetauscher ist beispielsweise ein Rohrbündelwärmetauscher, Doppelrohrwärmetauscher, Plattenwärmetauscher oder Spiralwärmetauscher. Bei Auslegungsdrücken für den Reaktor unter 100 bar wird bevorzugt ein Rohrbündelwärmetauscher verwendet, bei höheren Drücken wird bevorzugt ein oder mehrere hintereinander geschaltete Doppelrohrwärmetauscher verwendet.

Im oberen Bereich des Hydrierreaktors sind ein oder mehrere gas- und flüssigkeitsdurchlässige Einbauten (10) angebracht, durch den der obere Bereich vom unteren Bereich des Reaktors räumlich getrennt ist.
Als Einbauten kommen die voranstehend genannten Einbauten in Betracht, wobei die gas- und flüssigkeitsdurchlässigen Einbauten (10) bevorzugt Begasungsböden, wie Lochbleche, sind. Die Einbauten können auch wie obenstehend beschrieben als Füllkörper oder strukturierte Packungen ausgeführt sein.

Das Verhältnis von Gesamtlänge zu Durchmesser des Hydrierreaktors (1) liegt bevorzugt im Bereich von 5:1 bis 100:1, besonders bevorzugt 5:1 bis 50:1, ganz besonders bevorzugt 10:1 bis 30:1.
Die Ausführung eines Hydrierreaktors als langes, schlankes Rohr bietet mehrere Vorteile. Reaktionstechnisch hat sich erwiesen, dass ein langes Rohr vorteilhaft ist, da aufsteigende Gasblasen eine vergleichsweise lange Verweilzeit im Reaktor haben und somit mit hohem Umsatzgrad umgesetzt werden können, ohne wieder rückverdichtet werden zu müssen. Apparatetechnisch gesehen bietet eine derartige Reaktorausführung vor allem den Vorteil der einfachen Fertigung aus einem zylindrischen Rohr. Bei der Auslegung für hohe Systemdrücke reduziert sich außerdem die benötigte Wandstärke umso mehr, je geringer der Durchmesser ist.

Das Verhältnis des Volumens des oberen Bereichs des Reaktors, in dem die Einbauten angebracht sind und in dem die zweite Stufe des Hydrierverfahrens durchgeführt wird, zum Volumens des unteren Bereichs des Reaktors, in dem die erste Stufe des Verfahrens durchgeführt wird, liegt bevorzugt im Bereich von 0,05:1 bis 10:1, besonders bevorzugt im Bereich von 0,3:1 bis 2:1, ganz besonders bevorzugt im Bereich von 0,4:1 bis 1,5:1, insbesondere 1:1.

Das Volumen des oberen Bereichs des Reaktors, in dem die zweite Verfahrensstufe durchgeführt wird, wird durch die Begasungsböden in Einzelsegmente (Kompartments) unterteilt, wobei bevorzugt das Volumen eines durch die Begasungsböden unterteilten Einzelsegments im Bereich von 5 bis 50%, bezogen auf das Gesamtvolumen des Reaktors, beträgt.
Die Anzahl der Einzelsegmente beträgt vorzugsweise 1 bis 20, besonders bevorzugt 1 bis 10, insbesondere 2 bis 6.

In einer ganz besonders bevorzugten Ausführungsform liegt das Verhältnis des Volumens des oberen Bereichs des Reaktors, in dem die Einbauten angebracht sind und in dem die zweite Stufe des Hydrierverfahrens durchgeführt wird, zum Volumens des unteren Bereichs des Reaktors, in dem die erste Stufe des Verfahrens durchgeführt wird, im Bereich von 0,4:1 bis 1,5:1, insbesondere 1:1 bei einer Anzahl von 2 bis 6 Segmenten. Diese bevorzugte Ausführungsform ermöglicht eine ausreichende Verweilzeit zum Erzielen von hohen Umsätzen, auch bei Umsatz- und Lastschwankungen in der ersten Reaktionsstufe.

Im oberen Bereich des Reaktors befindet sich üblicherweise eine Austrittsstelle für das Reaktionsgemisch, das in einem Abscheider (11) in einen Flüssigkeitsstrom/Produktstrom (12) und einem Gastrom aufgetrennt wird. Der Gasstrom, welcher üblicherweise Wasserstoff enthält, kann durch eine Kreisgasleitung (9) der Mischdüse (3) zugeführt werden oder als Abgasstrom (20) der Aufarbeitung zugeführt werden.

Auf den beiliegenden Zeichnungen ist eine bevorzuge Ausführungsform des Hydrierreaktors sowie einer bevorzugten Ausführungsform der Mischdüse (3) dargestellt:

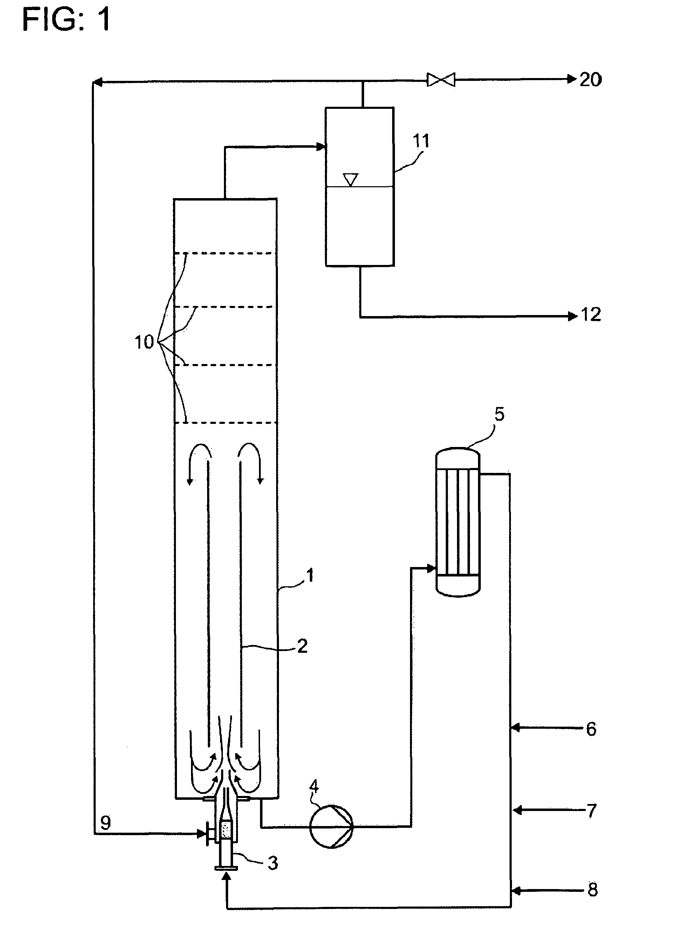

Figur 1 zeigt den kompletten Aufbau eines bevorzugten Hydrierreaktors, in dem Schlaufenreaktor und Blasensäulenreaktor in einem Apparat integriert sind, wobei dessen Aufbau und Funktionsweise im Folgenden erläutert wird:

Der Hydrierreaktor (1) ist als langes, zylindrisches Rohr ausgeführt (hochzylindrische Bauform), wobei wie voran stehend beschrieben, das Verhältnis von Gesamtlänge zu Durchmesser im Bereich von 5:1 bis 100:1,bevorzugte 5:1 bis 50:1, besonders bevorzugt 10:1 bis 30:1, beträgt. Der Reaktor ist mittels mehrerer Lochbleche (10), im gezeigten Beispiel vier Stück, so aufgeteilt, dass das Volumen im Bereich des Schlaufenreaktors (unterhalb des tiefsten Bodens) ca. 50% beträgt und die Volumina in den sich ergebenden oberen Kammern (Bereich des Blasensäulenreaktors) jeweils gleich groß sind, im gezeigten Beispiel jeweils 12,5%. Es ist jedoch auch, je nach Reaktionssystem und freigesetzter Reaktionswärme, eine andere Verteilung der einzelnen Segmente möglich.

Im unteren Bereich des Reaktors befindet sich ein zentrisch angebrachtes Rohr, das so genannte Leitrohr (2). Im unteren Boden des Reaktors ist eine Begasungsdüse (3) zentrisch angebracht, welche in das Leitrohr (2) hineinragt.
Aus dem unteren Teil des Reaktors wird mittels einer Umwälzpumpe (4) Flüssigkeit abgezogen und zur Kühlung durch einen Wärmetauscher (5) geführt. Nach dem Wärmetauscher wird Wasserstoffgas (6) sowie flüssiges Edukt (7) und flüssige Katalysatorsuspension (8) zur dieser Kreislaufflüssigkeit hinzugefügt. Die erzeugte Mischung wird schließlich über die Mischdüse (3) wieder dem Reaktor zugeführt. In der Mischdüse (3) wird Kreisgas (9) angesaugt, verdichtet und in der Flüssigkeit dispergiert.
Im oberen Teil der Mischdüse (3) wird Flüssigkeit aus dem Ringraum zwischen Leitrohr (2) und Außenmantel des Reaktors (1) angesaugt und mit der Flüssigkeits-Gas-Mischung weiter vermischt.
Oberhalb des Leitrohrs (2) trennt sich Flüssigkeit und Gas. Der Hauptanteil der Flüssigkeit strömt außerhalb des Leitrohrs wieder nach unten, der überschüssige Anteil der Flüssigkeit gelangt zusammen mit dem Gas durch die Lochbleche (10) nach oben in die einzelnen Segmente des Blasensäulenreaktors.
Am Kopf des Reaktors tritt die Flüssigkeits-Gas-Mischung aus und wird im Abscheidebehälter (11) in eine flüssige und eine gasförmige Phase getrennt. Die gasförmige Phase wird ganz oder teilweise der Mischdüse (3) über eine Kreisgasleitung (9) zugeführt oder aus dem Reaktorsystem entfernt (20). Die flüssige Phase wird aus dem Reaktorsystem komplett abgezogen und der Aufarbeitung zugeführt (12).

Figur 2 zeigt eine bevorzugte Ausführungsform einer Mischdüse (3).
Unten tritt Flüssigkeit in die Mischdüse ein (13), welche zunächst über einen Drallkörper (15) in Rotation versetzt wird und dann in der ersten Düse (16) mit einer Geschwindigkeit von vorzugsweise 10 bis 70 m/s in den zweiten Raum (17) eintritt. In diesem wird Kreisgas (14) durch den Flüssigkeitsstrahl angesaugt, verdichtet und mit der Flüssigkeit intensiv vermischt. Die Gas-Flüssig-Mischung tritt über eine zweite Düse in den Reaktor ein, womit Flüssigkeit aus dem Reaktor (18) angesaugt, zusammen mit der Gas-Flüssig-Mischung in das Expansionsrohr (19) und schließlich in das Leitrohr (2) des Reaktor gefördert wird.

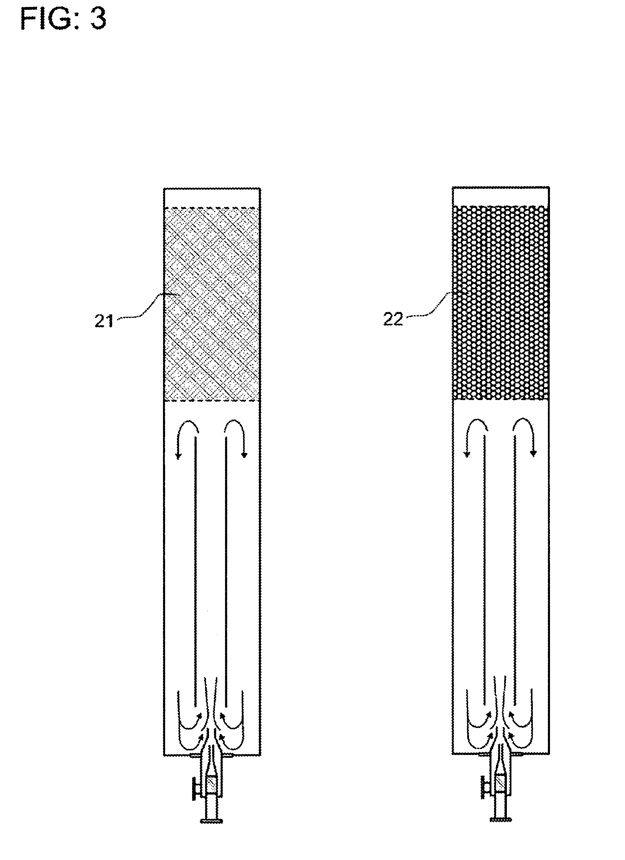

In Figur 3 sind zwei weitere Ausführungsformen dargestellt, in denen gezeigt wird, wie im Blasensäulenreaktor oberhalb des Schlaufenreaktors die Rückvermischung in vertikaler Richtung begrenzt werden kann. Anstelle der in Figur 1 gezeigten Lochbleche kann das gesamte Volumen des Blasensäulenreaktors mit strukturierter Packung (21) oder mit Füllkörpern (22) ausgefüllt werden.

Die vorliegenden Erfindung stellt ein technisch einfaches Verfahren zur Hydrierung von organischen Verbindungen dar, mit welchem auch stark exotherme Hydrierungen in einem engen Temperaturbereich und mit sehr hohem Endumsatz in kontinuierlicher Fahrweise durchgeführt werden können. Durch das erfindungsgemäße Verfahren wird es ermöglicht, die Reaktion in einem definierten Temperaturbereich auszuführen um somit gezielt das Isomerenverhältnis der Edukte zu steuern sowie die Bildung von unerwünschten Nebenprodukten zu vermeiden. Das Verfahren ermöglicht einen hohen Umsatz, so dass die Abtrennung von nicht umgesetzten Edukten und gebildeten Edukten vereinfacht wird. Weiterhin sind die Reaktionsführung und die apparative Umsetzung des Verfahrens technisch einfach und ökonomisch zu realisieren.
Aus dem in Figur 1 gezeigten Ausführungsbeispiel ist ersichtlich, dass vor allem der Hydrierreaktor (1) vollkommen ohne technisch aufwändige Einrichtungen wie beispielsweise Rührer auskommt. Durch die einfache Bauart ist diese Art von Reaktor insbesondere für Reaktionen bei hohem Druck besonders bevorzugt einsetzbar. Weiterhin ermöglicht dieses Verfahren lange Standzeiten, da die Anzahl von unerwünschten Abschaltungen des Reaktors zum Austausch oder der Regeneration des Katalysators verringert werden können, da dem Verfahren bei Verringerung der Aktivität bzw. Selektivität frischer Katalysator zugefügt werden kann und benutzter Katalysator aus dem Verfahren ausgeschleust werden kann.

Das erfindungsgemäße Verfahren wird durch die nachfolgenden Beispiele näher erläutert.

### Beispiel 1:

Im Beispiel wird eine kontinuierlich durchgeführte Hydrierung entsprechend dem erfindungsgemäßen Verfahren dargelegt.

Die Herstellung des verwendeten Katalysators, Ru-(IV)-oxidhydrat, wurde entsprechend Beispiel 1 in DE 21 32 547 durchgeführt, wobei lediglich auf die abschließende Trocknung im Vakuum verzichtet wurde. Zur Zubereitung der Katalysator-Suspension wurden 5500 g des so hergestellten Filterkuchens (mit 579 g Ru berechnet als 100% elementares Ruthenium) in einen Rührbehälter gegeben und mit 150 kg einer flüssigen Produktmischung (Austrag des Hydrierreaktors) intensiv vermischt, so dass die erzeugte Suspension 0,37 Gew.-% Ru enthielt.

Als Einsatzstoff für die Hydrierung wurde eine Schmelze von Bis(4-aminophenyl)methan (4,4'-MDA) mit einer Reinheit von ca. 99% eingesetzt. Das Edukt wurde als Schmelze (Schmelzpunkt ca. 90 °C) ohne weitere Verdünnung durch ein Lösungsmittel eingesetzt.

Die Hydrierung wurde in einer kontinuierlich betriebenen Produktionsanlage durchgeführt. Der Reaktor bestand aus einem senkrecht stehenden, zylindrischen Hochdruckrohr mit 600 mm Durchmesser und einer Gesamthöhe von 16 m. Im oberen Teil des Reaktors waren vier Lochbleche im Abstand von jeweils 2 m angebracht. Die Lochbleche hatten jeweils 36 Löchern von 10 mm Durchmesser. Der oberste Boden befand sich 2 m unterhalb des oberen Reaktordeckels. Im unteren Teil des Reaktors war ein Leitrohr mit einer Länge von 6,3 m und einem Durchmesser von 0,35 m zentrisch eingebaut, wobei die Oberkante dieses Rohres 1 m unterhalb des untersten Lochbleches angebracht war. Am Boden des Reaktors war eine mehrstufige Düse entsprechend Figur 2 zentrisch eingebaut. Die erste Düse hatte einen Durchmesser von 15 mm; die zweite Düse von 33 mm. Das Expansionsrohr hatte an der engsten Stelle einen Durchmesser von 40 mm, am Expansionsrohraustritt von 80 mm und das Expansionsrohr besaß eine Länge von 715 mm, gemessen von der Spitze der zweiten Düse. Das Expansionsrohr ragte ca. 550 mm in das Leitrohr hinein.
Bei der Durchführung der Hydrierung werden mit einer Kreiselpumpe ca. 25 m³/h an Flüssigkeit aus dem untersten Teils des Reaktor abgezogen, mittels Wärmetauscher abgekühlt und nach Zugabe von reinem Wasserstoff, an flüssigem Edukt und von Katalysatorlösung der Düse (Anschluss 13) zugeführt. Die erste Düse (16) erzeugte einen Unterdruck und zog dadurch Gas aus dem Abscheider (11) an. Die so angesaugte Menge an Kreisgas betrug ca. 5.000 Nm³/h.

Dem Reaktor wurden kontinuierlich 500 kg/h einer Schmelze von 4,4'-MDA mit einer Temperatur von 120 °C sowie 6,7 kg/h der Katalysatorsuspension zugeführt. Der Druck im Reaktor wurde durch Nachführung von reinem Wasserstoff in den Umpumpkreislauf auf 200 bar geregelt. Die Temperatur im Reaktor betrug 230 °C am Boden des Reaktors, 233 °C unterhalb des untersten Lochbleches und 238°C am obersten Punkt des Reaktors. Das oben austretende Gas-Flüssigkeitsmischung wurde in einem Abscheider getrennt. Die anfallende Flüssigkeit wurde standgeregelt abgezogen, entspannt und auf 80 °C abgekühlt. Das im Abscheider anfallende Gas wurde vollständig in einer Menge von 5.000 Nm³/h wieder der Mischdüse und dem unteren Teil des Reaktors zugeführt.

Die Analyse der flüssigen Produktmischung mittels Gaschromatographie ergab folgende Werte (Konzentrationsangaben in Gew.-%):

| | Analyse Reaktionsaustrag Gew.-% |
|---|---|
| Leichtsiedende Nebenprodukte | 6,85 |
| Hochsiedende Nebenprodukte | 3 |
| Endprodukt | 90 |
| Zwischenstufe | 0,1 |
| Ausgangsprodukt | 0,05 |

| | |
|---|---|
| Endprodukt: Bis-(4-amino-cyclohexyl)-methan Zwischenstufe: (4-Amino-cyclohexyl)-(4-amino-phenyl)-methan Ausgangsprodukt: Bis(4-amino-phenyl)methan | |

Der Umsatz des Einsatzstoffes lag dabei bei 99,95% mit einer Selektivität bezüglich des Endproduktes von ca. 90%.

### Beispiel 2:

Als Reaktor wurde das in Beispiel 1 beschriebene Reaktorsystem verwendet.

Als Einsatzstoff für die Hydrierung wurde eine Schmelze von Bis(4-amino-3-methylphenyl)methan ("o-Toluidinbase") mit einer Reinheit von ca. 99% eingesetzt. Das Edukt wurde als Schmelze (Schmelzpunkt ca. 160 °C) ohne weitere Verdünnung durch ein Lösungsmittel eingesetzt.

Als Katalysators wurde Ru-(IV)-oxidhydrat, hergestellt entsprechend Beispiel 1 in DE 21 32 547, eingesetzt. Der Katalysator wurde wiederum in flüssigem Reaktoraustrag suspendiert, so dass sich eine Ru-Konzentration von 0,37 Gew.-% in dieser Suspension ergab.

Dem Reaktor wurden kontinuierlich 750 kg/h einer Schmelze von o-Toluidinbase mit einer Temperatur von 180 °C sowie 10 kg/h der Katalysatorsuspension zugeführt. Der Druck im Reaktor wurde durch Nachführung von reinem Wasserstoff in den Umpumpkreislauf auf 200 bar geregelt. Die Temperatur im Reaktor betrug 240 °C am Boden des Reaktors, 245°C unterhalb des untersten Lochbleches und 248 °C am obersten Punkt des Reaktors. Das oben austretende Gas-Flüssigkeitsmischung wurde in einem Abscheider getrennt. Die anfallende Flüssigkeit wurde standgeregelt abgezogen, entspannt und auf 80 °C abgekühlt. Das im Abscheider anfallende Gas wurde vollständig in einer Menge von 5.000 Nm³/h wieder der Mischdüse und dem unteren Teil des Reaktors zugeführt.

Die Analyse der flüssigen Produktmischung mittels Gaschromatographie ergab folgende Werte (Konzentrationsangaben in Gew.-%):

| | Analyse Reaktionsaustrag Gew.-% |
|---|---|
| Leichtsiedende Nebenprodukte | 3,5 |
| Hochsiedende Nebenprodukte | 1,3 |
| Endprodukt | 95,1 |
| Zwischenstufe | 0,08 |
| Ausgangsprodukt | 0,02 |

| | |
|---|---|
| Endprodukt: Bis-(4-amino-3-methylcyclohexyl)-methan Zwischenstufe: (4-Amino-3-methylcyclohexyl)-(4-amino-3-methylphenyl)-methan Ausgangsprodukt: Bis(4-amino-3-methylphenyl)methan | |

Der Umsatz des Einsatzstoffes lag dabei bei >99,9% mit einer Selektivität bezüglich des Endproduktes von ca. 95%.

## Patentansprüche

1. Kontinuierliches Verfahren zur Hydrierung organischer Verbindungen in einem mehrphasigen System in Gegenwart eines homogenen oder heterogenen Katalysators, **dadurch gekennzeichnet, dass** man das Verfahren in zwei Stufen durchführt, wobei die erste Stufe in einem Schlaufenreaktor mit externem Wärmetauscher und die zweite Stufe in einem Blasensäulenreaktor mit begrenzter Rückmischung durchgeführt wird.

2. Verfahren nach Anspruch 1, **dadurch gekennzeichnet, dass** man die Hydrierung in Gegenwart eines heterogenen Katalysators durchführt.

3. Verfahren nach mindestens einem der Ansprüche 1 bis 2, **dadurch gekennzeichnet, dass** man als organische Verbindungen aromatische Verbindungen einsetzt.

4. Verfahren nach Anspruch 3, **dadurch gekennzeichnet, dass** man als aromatische Verbindungen Polymer-MDA, Anilin, 2,4-Diaminotoluol, 2,6-Diaminotoluol, o-Phenylendiamin, m-Phenylendiamin, p-Phenylendiamin, Bis-(4-aminophenyl)-methan (MDA), Meta-Xylendiamin (MXDA), Bis-(4-amino-3-methylphenyl)-methan und/oder Bis-(4-amino-3,5-dimethylphenyl)-methan einsetzt.

5. Verfahren nach mindestens einem der Ansprüche 1 bis 4, **dadurch gekennzeichnet, dass** man die erste Stufe des Verfahrens in einem Schlaufenreaktor mit interner Schlaufenströmung durchführt.

6. Verfahren nach mindestens einem der Ansprüche 1 bis 5, **dadurch gekennzeichnet, dass** die Rückmischung im Blasensäulenreaktor der zweiten Stufe durch Einbauten begrenzt wird.

7. Verfahren nach Anspruch 6, **dadurch gekennzeichnet, dass** die Einbauten gas- und flüssigkeitsdurchlässige Begasungsböden darstellen.

8. Verfahren nach mindestens einem der Ansprüche 6 bis 7, **dadurch gekennzeichnet, dass** der Blasensäulenreaktor durch die Begasungsböden in mehrere Einzelsegmente unterteilt wird und die Anzahl der Einzelsegmente 1 bis 20 beträgt.

9. Verfahren nach Anspruch 6, **dadurch gekennzeichnet, dass** die Einbauten Füllkörper oder strukturierte Packungen darstellen.

10. Verfahren nach mindestens einem der Ansprüche 1 bis 9, **dadurch gekennzeichnet, dass** die zwei Stufen des Verfahrens in einem Apparat angeordnet sind.

11. Verfahren nach mindestens einem der Ansprüche 1 bis 10, **dadurch gekennzeichnet, dass** zwischen der Eduktzuführung im Schlaufenreaktors der ersten Verfahrensstufe und dem Austritt des Gas-Flüssigkeitsgemisches im Blasensäulenreaktor der zweiten Verfahrensstufe eine maximale Temperaturdifferenz von 20 K auftritt.

12. Verfahren nach mindesten einem der Ansprüche 1 bis 11, **dadurch gekennzeichnet, dass** der Umsatz im Schlaufenreaktor 92 bis 98% beträgt.

13. Verfahren zur Herstellung von Tensiden, Arznei- und Pflanzenschutzmitteln, Stabilisatoren, Lichtschutzmitteln, Polymeren, Polyamiden, Isocyanaten, Härtern für Epoxyharze, Katalysatoren für Polyurethane, Zwischenprodukten zur Herstellung quaternärer Ammoniumverbindungen, Weichmachern, Korrosionsinhibitoren, Kunstharzen, lonenaustauschem, Textilhilfsmitteln, Farbstoffen, Vulkanisationsbeschleunigern, Emulgatoren und/oder Ausgangssubstanzen für die Herstellung von Harnstoffen und Polyharnstoffen, **dadurch gekennzeichnet, dass** man in einem ersten Schritt ein cycloaliphatisches Amin durch ein Verfahren gemäß mindestens einem der Ansprüche 1 bis 12 herstellt, und in einem zweiten Schritt das in Schritt 1 hergestellte cycloaliphatische Amin in einem Verfahren zur Herstellung von Tensiden, Arznei- und Pflanzenschutzmitteln, Stabilisatoren, Lichtschutzmitteln, Polymeren, Polyamiden, Isocyanaten, Härtern für Epoxyharze, Katalysatoren für Polyurethane, Zwischenprodukten zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern, Emulgatoren und/oder Ausgangssubstanzen für die Herstellung von Harnstoffen und Polyharnstoffen einsetzt.

## Claims

1. A continuous process for hydrogenating organic compounds in a polyphasic system in the presence of a homogeneous or heterogeneous catalyst, which comprises performing the process in two stages, the first stage being performed in a loop reactor with an external heat exchanger and the second stage in a bubble column reactor with limited backmixing.

2. The process according to claim 1, wherein the hydrogenation is performed in the presence of a heterogeneous catalyst.

3. The process according to at least one of claims 1 and 2, wherein the organic compounds used are aromatic compounds.

4. The process according to claim 3, wherein the aromatic compounds used are polymeric MDA, aniline, 2,4-diaminotoluene, 2,6-diaminotoluene, o-phenylenediamine, m-phenylenediamine, p-phenylenediamine, bis(4-aminophenyl)methane (MDA), meta-xylenediamine (MXDA), bis(4-amino-3-methylphenyl)methane and/or bis(4-amino-3,5-dimethylphenyl)methane.

5. The process according to at least one of claims 1 to 4, wherein the first stage of the process is performed in a loop reactor with internal loop flow.

6. The process according to at least one of claims 1 to 5, wherein the backmixing in the bubble column reactor of the second stage is restricted by internals.

7. The process according to claim 6, wherein the internals are gas- and liquid-permeable sparging trays.

8. The process according to at least one of claims 6 and 7, wherein the bubble column reactor is divided into several individual segments by the sparging trays and the number of individual segments is from 1 to 20.

9. The process according to claim 6, wherein the internals are random packings or structured packings.

10. The process according to at least one of claims 1 to 9, wherein the two stages of the process are arranged in one apparatus.

11. The process according to at least one of claims 1 to 10, wherein a maximum temperature difference of 20 K occurs between the reactant feed in the loop reactor of the first process stage and the exit of the gas-liquid mixture in the bubble column reactor of the second process stage.

12. The process according to at least one of claims 1 to 11, wherein the conversion in the loop reactor is from 92 to 98%.

13. A process for producing surfactants, medicaments and crop protection compositions, stabilizers including light stabilizers, polymers, polyamides, isocyanates, hardeners for epoxy resins, catalysts for polyurethanes, intermediates for preparing quaternary ammonium compounds, plasticizers, corrosion inhibitors, synthetic resins, ion exchangers, textile assistants, dyes, vulcanization accelerants, emulsifiers and/or starting substances for the preparation of ureas and polyureas, which comprises, in a first step, preparing a cycloaliphatic amine by a process according to at least one of claims 1 to 12, and, in a second step, using the cycloaliphatic amine prepared in step 1 in a process for producing surfactants, medicaments and crop protection compositions, stabilizers including light stabilizers, polymers, polyamides, isocyanates, hardeners for epoxy resins, catalysts for polyurethanes, intermediates for preparing quaternary ammonium compounds, plasticizers, corrosion inhibitors, synthetic resins, ion exchangers, textile assistants, dyes, vulcanization accelerants, emulsifiers and/or starting substances for the preparation of ureas and polyureas.

## Revendications

1. Procédé continu pour hydrogéner des composés organiques dans un système à plusieurs phases en présence d'un catalyseur homogène ou hétérogène, **caractérisé en ce qu'**on réalise le procédé en deux étapes, la première étape étant réalisée dans un réacteur à boucle équipé d'un échangeur de chaleur externe et la deuxième étape étant réalisée dans un réacteur à colonne à bulles avec un mélange en retour limité.

2. Procédé selon la revendication 1, **caractérisé en ce qu'**on réalise l'hydrogénation en présence d'un catalyseur hétérogène.

3. Procédé selon au moins l'une quelconque des revendications 1 à 2, **caractérisé en ce qu'**on utilise, comme composés organiques, des composés aromatiques.

4. Procédé selon la revendication 3, **caractérisé en ce qu'**on utilise, comme composés aromatiques, du polymère-MDA, de l'aniline, du 2,4-diaminotoluène, du 2,6-diaminotoluène, de l'o-phénylènediamine, du m-phénylènediamine, du p-phénylènediamine, du bis-(4-aminophényl)-méthane (MDA), de la méta-xylènediamine (MXDA), du bis-(4-amino-3-méthylphényl)-méthane et/ou du bis-(4-amino-3,5-diméthylphényl)-méthane.

5. Procédé selon au moins l'une quelconque des revendications 1 à 4, **caractérisé en ce qu'**on réalise la première étape du procédé dans un réacteur à boucle avec un écoulement en boucle interne.

6. Procédé selon au moins l'une quelconque des revendications 1 à 5, **caractérisé en ce que** le mélange en retour dans le réacteur à colonne à bulles de la deuxième étape est limité par des encastrements.

7. Procédé selon la revendication 6, **caractérisé en ce que** les encastrements sont des plateaux de gazage perméables aux gaz et aux liquides.

8. Procédé selon au moins l'une quelconque des revendications 6 à 7, **caractérisé en ce que** le réacteur à colonne à bulles est réparti en plusieurs segments individuels par les plateaux de gazage et le nombre de segments individuels est de 1 à 20.

9. Procédé selon la revendication 6, **caractérisé en ce que** les encastrements représentent des corps de remplissage ou des garnissages structurés.

10. Procédé selon au moins l'une quelconque des revendications 1 à 9, **caractérisé en ce que** les deux étapes du procédé sont disposées dans un appareil.

11. Procédé selon au moins l'une quelconque des revendications 1 à 10, **caractérisé en ce qu'**entre l'alimentation en produits de départ dans le réacteur à boucle de la première étape de procédé et la sortie du mélange gaz-liquide dans le réacteur à colonne à bulles de la deuxième étape de procédé, la différence de température maximale est de 20 K.

12. Procédé selon au moins l'une quelconque des revendications 1 à 11, **caractérisé en ce que** la conversion dans le réacteur à boucle est de 92 à 98%.

13. Procédé pour la préparation d'agents tensioactifs, de médicaments et d'agents de phytoprotection, de stabilisateurs, d'agents de protection contre la lumière, de polymères, de polyamides, d'isocyanates, de durcisseurs pour les résines époxy, de catalyseurs pour les polyuréthanes, de produits intermédiaires pour la préparation de composés d'ammonium quaternaire, de plastifiants, d'inhibiteurs de corrosion, de résines artificielles, d'échangeurs ioniques, d'adjuvants pour textiles, de colorants, d'accélérateurs de vulcanisation, d'émulsifiants et/ou de substances de départ pour la préparation d'urées et de polyurées, **caractérisé en ce qu'**on prépare, dans une première étape, une amine cycloaliphatique par un procédé selon au moins l'une quelconque des revendications 1 à 12 et on utilise dans une deuxième étape l'amine cycloaliphatique préparée dans l'étape 1 dans un procédé pour la préparation d'agents tensioactifs, de médicaments et d'agents de phytoprotection, de stabilisateurs, d'agents de protection contre la lumière, de polymères, de polyamides, d'isocyanates, de durcisseurs pour les résines époxy, de catalyseurs pour les polyuréthanes, de produits intermédiaires pour la préparation de composés d'ammonium quaternaire, de plastifiants, d'inhibiteurs de corrosion, de résines artificielles, d'échangeurs ioniques, d'adjuvants pour textiles, de colorants, d'accélérateurs de vulcanisation, d'émulsifiants et/ou de substances de départ pour la préparation d'urées et de polyurées.
